# EUROPÄISCHE PATENTSCHRIFT

(11) **EP 0 653 418 B1**
(45) Veröffentlichungstag und Bekanntmachung des Hinweises auf die Patenterteilung: **05.03.1997**
(21) Anmeldenummer: 94117414.6
(22) Anmeldetag: 04.11.1994
(51) Int. Cl.: C07C 233/60, C07C 271/40, C07C 271/42, C07C 271/58, A01N 37/24, A01N 47/06

(54) **P-Hydroxyanilinderivate, Verfahren zu ihrer Herstellung und ihre Verwendung zur Bekämpfung von Schadpilzen oder Schädlingen**
P-hydroxy-aniline derivatives, process for their preparation and their use as fungicides or pesticides
Dérivés de para-hydroxy-aniline, procédé pour leur préparation et leur utilisation comme fongicides ou pesticides

(30) Priorität: 11.11.1993 DE 4338512; 26.05.1994 DE 4418380
(43) Veröffentlichungstag der Anmeldung: 17.05.1995
(73) Patentinhaber: BASF Aktiengesellschaft, 67063 Ludwigshafen (DE)
(72) Erfinder: Wagner, Oliver, Dr., D-66450 Bexbach (DE); Eicken, Karl, Dr., D-67157 Wachenheim (DE); Götz, Norbert, Dr., D-67547 Worms (DE); Rang, Harald, Dr., D-67122 Altrip (DE); Ammermann, Eberhard, Dr., D-64646 Heppenheim (DE); Lorenz, Gisela, Dr., D-67434 Neustadt (DE)

(56) Entgegenhaltungen:
- EP-A- 0 339 418
- EP-A- 0 416 359
- EP-A- 0 416 365
- EP-A- 0 520 252
- DE-A- 2 354 873
- US-A- 3 636 094

## Beschreibung

Die vorliegende Erfindung betrifft p-Hydroxyanilinderivate der Formel I 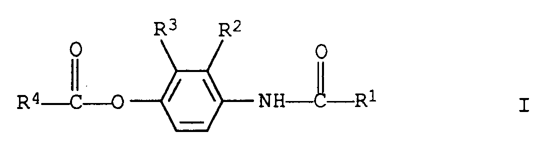 in der die Substituenten die folgende Bedeutung haben:
- R¹: C₆-C₁₅-Bicycloalkyl oder C₇-C₁₅-Bicycloalkenyl, wobei diese Gruppen partiell oder vollständig halogeniert sein können und/oder ein bis fünf der folgenden Reste tragen können: C₁-C₄-Alkyl, C₁-C₄-Halogenalkyl, C₁-C₄-Alkoxy, C₁-C₄-Halogenalkoxy und Aryl, welches partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
- R² und R³: unabhängig voneinander Halogen, C₁-C₄-Alkyl, C₁-C₄-Halogenalkyl, C₁-C₄-Alkoxy und C₁-C₄-Halogenalkoxy;
- R⁴: C₁-C₆-Alkyl oder C₂-C₆-Alkenyl, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl, wobei diese Gruppen ein bis drei der folgenden Reste tragen können: Halogen, C₁-C₄-Alkyl, C₁-C₄-Halogenalkyl und C₁-C₄-Alkoxy;
Aryl, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
OR⁵ oder NR⁵R⁶, wobei
- R⁵: C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy;
oder Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio; und
- R⁶: Wasserstoff oder C₁-C₆-Alkyl bedeutet,
sowie deren Salze.

Außerdem betrifft die Erfindung Verfahren zur Herstellung der Verbindungen I, sie enthaltende Mittel und ihre Verwendung zur Herstellung derartiger Mittel sowie ihre Verwendung zur Bekämpfung von Schadpilzen oder Schädlingen.

Aus der Literatur sind Cycloalkylcarbonsäureanilide mit fungizider Wirkung bekannt (DE-A 40 12 712, DE-A 40 12 791, DE-A 41 20 904, EP-A 339 418, EP-A 416 359, EP-A 416 365). Bicycloalkyl bzw. Bicycloalkenyl carbonsäureanilid derivate mit fungizider Wirkung sind auch bekannt (DE-A-2354873, US-A-3636094).

Der vorliegenden Erfindung lagen neue Verbindungen mit verbesserter Wirkung gegen ein breiteres Spektrum von Schadpilzen als Aufgabe zugrunde.

Demgemäß wurden die eingangs definierten Verbindungen I gefunden.

Außerdem wurden Verfahren zur Herstellung der Verbindungen I, sie enthaltende Mittel, ihre Verwendung zur Herstellung derartiger Mittel sowie ihre Verwendung zur Bekämpfung von Schadpilzen oder Schädlingen gefunden.

Die Verbindungen I können im allgemeinen in an sich bekannter Weise ausgehend von den entsprechenden p-Hydroxyanilinen durch Veresterung der phenolischen OH-Gruppe und Amidierung der NH₂-Gruppe entsprechend dem folgenden Reaktionsschema hergestellt werden.

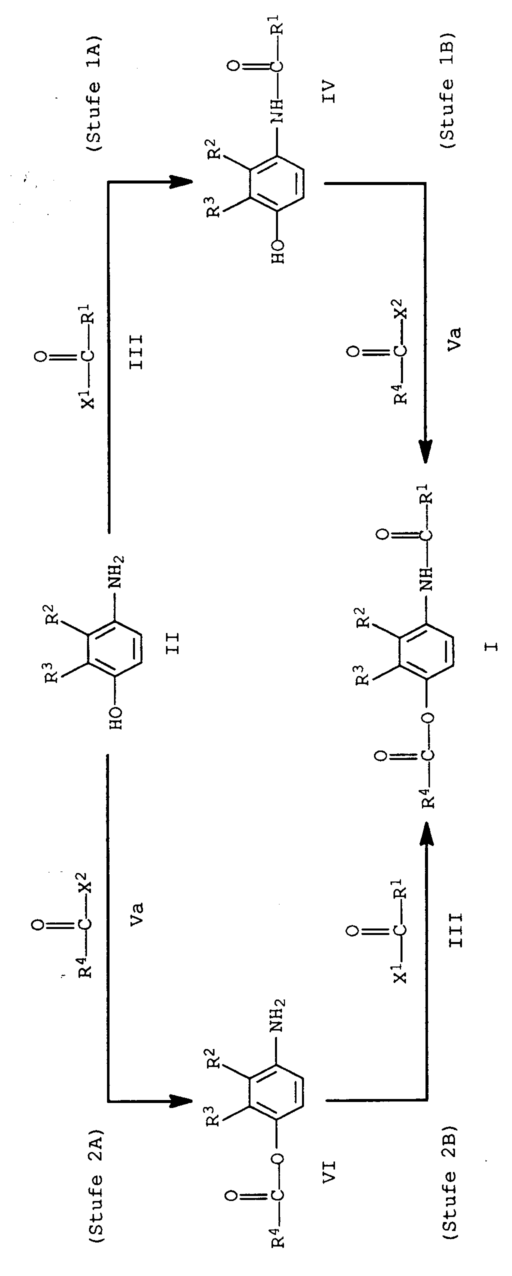

Die einzelnen Umsetzungsschritte werden üblicherweise wie folgt durchgeführt:

### Stufe 1A: Herstellung der Carbonsäureanilide IV

Man erhält die Carbonsäureanilide der Formel IV dadurch, daß man ein p-Hydroxyanilin der Formel II in an sich bekannter Weise (DE-A 32 02 100 (EP-A 339 418)) in einem inerten organischen Lösungsmittel in Gegenwart einer Base mit einem Carbonylderivat der Formel III umsetzt.

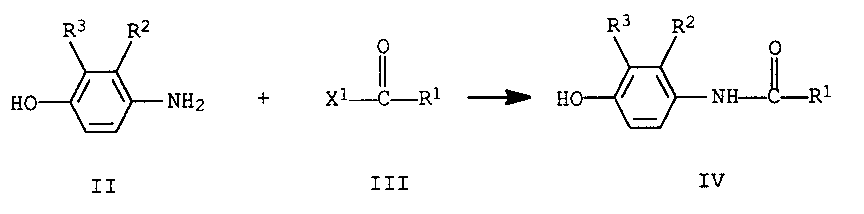

Die Variable X¹ in der Formel III steht für Halogen wie insbesondere Chlor, Brom und Jod, oder eine bei Acylierungsreaktionen gebräuchliche Abgangsgruppe, z.B. R¹-CO-O.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dioxan, Tetrahydrofuran und Dimethylformamid.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium; außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Alkalimetallcarbonate, -alkoholate und tertiäre Amine.

Die Basen werden im allgemeinen in äquimolaren Mengen eingesetzt, sie können aber auch im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, III in einem Über- oder Unterschuß bezogen auf II einzusetzen.

Die für die Umsetzung benötigten Ausgangsstoffe II und III sind aus der Literatur bekannt (II: J. chem. soc. PT I, 1, 1 (1973); Houben-Weyl, Vol. 10/1, S. 1140f; ibid. Vol. 6/1c, S. 85-101; III: Houben-Weyl, E5 Teil 1, S. 587) oder können gemäß der zitierten Literatur hergestellt werden.

### Stufe 1B: Herstellung der p-Hydroxyanilinderivate I aus den Carbonsäureaniliden IV

Man erhält die p-Hydroxyanilinderivate I durch Umsetzung eines Carbonsäureanilids der Formel IV mit einem Carbonylderivat der Formel Va.

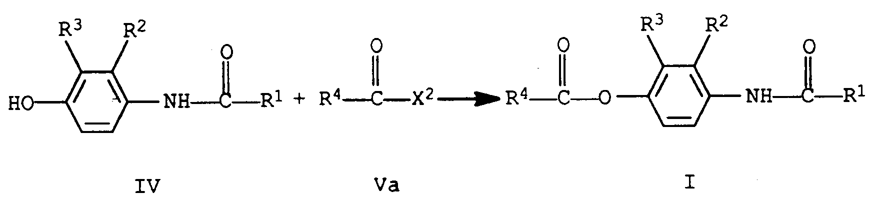

Die Variable X² in der Formel Va steht für Halogen wie insbesondere Chlor, Brom und Jod.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dichlormethan, Tetrahydrofuran, Aceton und Dimethylamid.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium; außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Alkalimetallcarbonate, -alkoholate und tertiäre Amine.

Die Basen werden im allgemeinen in äquimolaren Mengen eingesetzt, sie können aber auch im Überschuß oder gegebenenfalls als Lösungsmittel verwendet werden.

Die Verbindungen IV und Va werden im allgemeinen in äquimolaren Mengen miteinander umgesetzt. Es kann für die Ausbeute vorteilhaft sein, Va in einem Über- oder Unterschuß bezogen auf IV einzusetzen.

Die für die Umsetzung benötigten Ausgangsstoffe Va bzw. Vb sind aus der Literatur bekannt (Houben-Weyl, E4, S. 9; Houben-Weyl, E5, Teil 1, S. 587; Houben-Weyl, E4, S. 768) oder können gemäß der zitierten Literatur hergestellt werden.

Besonders bevorzugt erhält man Verbindungen I, in denen R⁴ für eine Gruppe NHR⁵ steht dadurch, daß man ein Carbonsäureamid der Formel IV in an sich bekannter Weise (Houben-Weyl, E4, S. 768) mit einem Isocyanat der Formel Vb hinsetzt.

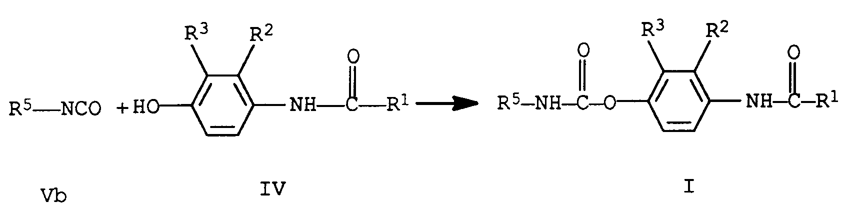

Diese Umsetzung erfolgt üblicherweise bei Temperaturen von -20°C bis 140°C, vorzugsweise -5°C bis 50°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Methylenchlorid und Dimethylformamid.

Es können auch Gemische der genannten Lösungsmittel verwendet werden.

Die Verbindungen IV und Vb werden im allgemeinen in äquimolaren Mengen miteinander umgesetzt. Es kann für die Ausbeute vorteilhaft sein, eine der Verbindungen in einem Über- oder Unterschuß bezogen auf die andere einzusetzen.

### Stufe 2A: Herstellung der Verbindungen VI

Man erhält die Verbindungen der Formel VI dadurch, daß man ein p-Hydroxyanilin der Formel II bzw. ein entsprechendes Phenolat in an sich bekannter Weise (Houben-Weyl, E4, S. 68) in einem inerten organischen Lösungsmittel in Gegenwart einer Base mit einem Carbonylderivat der Formel Va umsetzt.

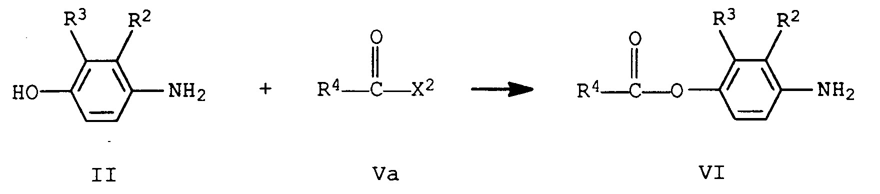

Die Variable X² in der Formel Va steht für Halogen wie insbesondere Chlor, Brom und Jod.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Aceton und Dimethylformamid.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylmin und N-Methylpiperidin, Pyridin, substituierte Pyridine wie Collidin, Lutidin und 4-Dimethylaminopyridin sowie bicyclische Amine in Betracht.

Besonders bevorzugt werden Alkalimetallcarbonate, Alkalimetallhydride und -alkoholate.

Die Basen werden im allgemeinen in äquimolaren Mengen eingesetzt, sie können aber auch im Überschuß oder gegebenenfalls als Lösungsmittel verwendet werden.

Die Verbindungen II und Va werden im allgemeinen in äquimolaren Mengen miteinander umgesetzt. Es kann für die Ausbeute vorteilhaft sein, Va in einem Über- oder Unterschuß bezogen auf II einzusetzen.

Besonders bevorzugt erhält man die Verbindungen VI, in denen R⁴ für eine Gruppe NHR⁵ steht, dadurch daß man ein p-Hydroxyanilin der Formel II bzw. das entsprechende Phenolat in an sich bekannter Weise (Houben-Weyl, E4, S. 768) mit einem Isocyanat der Formel Vb umsetzt.

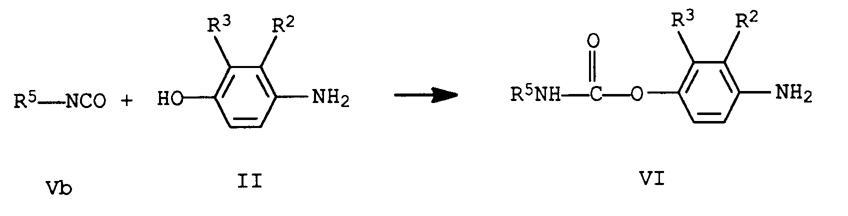

Diese Umsetzung erfolgt üblicherweise bei Temperaturen von -20°C bis 140°C, vorzugsweise -5°C bis 50°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Methylenchlorid, Dimethylformamid und Toluol.

Es können auch Gemische der genannten Lösungsmittel verwendet werden.

Die Verbindungen II und Vb werden im allgemeinen in äquimolaren Mengen miteinander umgesetzt. Es kann für die Ausbeute vorteilhaft sein, eine der Verbindungen in einem Über- oder Unterschuß bezogen auf die andere einzusetzen.

### Stufe 2B: Herstellung der p-Hydroxyanilinderivate I aus den Verbindungen VI

Man erhält die p-Hydroxyanilinderivate I durch Umsetzung einer Verbindung der Formel VI mit einem Carbonylderivat der Formel III.

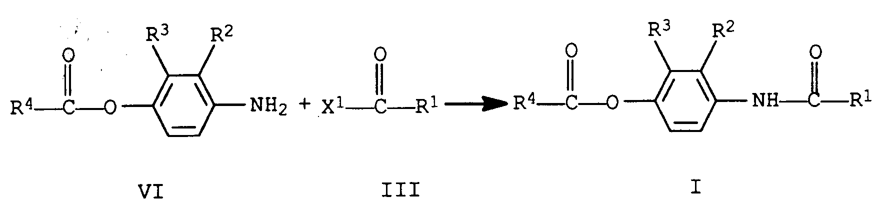

Die Variable X¹ in der Formel III steht für Halogen wie insbesondere Chlor, Brom und Jod, oder eine bei Acylierungsreaktionen gebräuchliche Abgangsgruppe, z.B. wie vorstehend bei Stufe 1A genannt.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Toluol, Tetrahydrofuran, Aceton und Dimethylsulfoxid.

Es können auch Gemische der genannten Lösungsmittel verwendet werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können ein oder mehrere Asymmetriezentren enthalten und werden nach den beschriebenen Verfahren in Form von Enantiomeren- oder Diastereomerengemischen erhalten. Die Mengenverhältnisse können dabei in Abhängigkeit von den Gruppen unterschiedlich sein. Diese Gemische können ggf. nach üblichen Methoden getrennt werden. Die Verbindungen I können sowohl als reine Isomere oder auch als Isomerengemische zur Anwendung kommen.

Die Verbindung I ist wegen des basischen Charakters der NH-Gruppierung in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Schwefelsäure, Phosphorsäure und Salpetersäure.

Als organischen Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Bei der eingangs angegebenen Definition der Verbindungen I wurden Sammelbegriffe verwendet, die allgemein repräsentativ für die folgenden Substituenten stehen:
Halogen: Fluor, Chlor, Brom und Jod;
Alkyl: geradkettige oder verzweigte Alkylgruppen mit 1 bis 3, 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl bzw. partiell oder vollständig halogeniertes Alkyl: geradkettige oder verzweigte Alkylgruppen mit 1 bis 4, 6 oder 8 Kohlenstoffatomen wie vorstehend genannt, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 3 oder 4 Kohlenstoffatomen, wie Methyloxy, Ethyloxy, Propyloxy, 1-Methylethyloxy, Butyloxy, 1-Methyl-propyloxy, 2-Methylpropyloxy und 1,1-Dimethylethyloxy;
Halogenalkoxy: geradkettige oder verzweigte partiell oder vollständig halogenierte Alkylgruppen mit 1 bis 4 Kohlenstoffatomen wie vorstehend genannt, wobei diese Gruppen über ein Sauerstoffatom (-O-) an das Gerüst gebunden sind;
Alkylthio: geradkettige oder verzweigte Alkylgruppen mit 1 bis 4 Kohlenstoffatomen wie vorstehend genannt, welche über ein Schwefelatom (-S-) an das Gerüst gebunden sind;
Alkenyl: geradkettige oder verzweigte Alkenylgruppen mit 2 bis 6 Kohlenstoffatomen und einer Doppelbindung in einer beliebigen Position, z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl3-butenyl, 2,3-Dimethyl-1-butenyl, 2,3-Dimethyl-2-butenyl, 2,3-Dimethyl-3- butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl- 2-propenyl, 1-Ethyl-1-methyl-2-propenyl, 1-Ethyl-2-methyl-1-propenyl und 1-Ethyl-2-methyl-2-propenyl;
Cycloalkyl: monocyclische Alkylgruppen mit 3 bis 7 Kohlenstoffringgliedern: Cyclopropyl, Cyclobutyl, Cyclopentyl, Cyclohexyl, und Cycloheptyl;
Cycloalkenyl: monocyclische Alkenylgruppen mit 5 bis 7 Kohlenstoffringgliedern und einer oder zwei Doppelbindungen: 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl;
Bicycloalkyl: bicyclische Alkylgruppen mit 6 bis 15 Kohlenstoffringgliedern, z.B. Bicyclo-[2.1.1]-hex-5-yl, Bicyclo-[2.2.1]-hept-2-yl, Bicyclo-[2.2.2]oct-2-yl, Bicyclo-[3.2.1]-oct-6-yl, Bicyclo-[3.2.2]-non-6-yl, Bicyclo-[4.2.2]-dec-7-yl, Bicyclo-[3.1.0]-hex-1-yl, Bicyclo-[4.1.0]-hept-1-yl, Bicyclo-[4.3.0]-non-1-yl, Bicyclo-[4.4.0]-dec-1-yl, besonders bevorzugt 5-Methyl-bicyclo-[2.1.1]-hex-5-yl, 2-Methyl-bicyclo-[2.2.1]hept-2-yl, 2-Methyl-bicyclo-[2.2.2]oct-2-yl, 6-Methyl-bicyclo-[3.2.1]-oct-6-yl, 6-Methyl-bicyclo-[3.2.2]-non-6-yl, 7-Methyl-bicyclo-[4.2.2]-dec-7-yl, 1-Methyl-bicyclo-[3.1.0]-hex-1-yl, 1-Methyl-bicyclo-[4.1.0]-hept-1-yl, 1-Methyl-bicyclo-[4.3.0]-non-1-yl, 1-Methyl-bicyclo-[4.4.0]-dec-1-yl, 2-Methyl-bicyclo-[3.1.0]-hex-1-yl, 2-Methyl-bicyclo-[4.1.0]-hept-1-yl, 2-Methyl-bicyclo-[4.3.0]-non-1-yl, 2-Methyl-bicyclo-[4.4.0]-dec-1-yl;
Bicycloalkenyl: bicyclische Alkenylgruppen mit 7 bis 15 Kohlenstoffringgliedern, z.B. Bicyclo-[2.2.1]-hept-2-en-5-yl, Bicyclo-[2.2.2]-oct-2-en-5-yl, Bicyclo-[4.2.2]-dec-7-en-2-yl, Bicyclo-[4.3.0]-non-7-en-1-yl, Bicyclo-[4.4.0]-dec-3-en-1-yl, Bicyclo-[4.1.0]-hept-3-en-1-yl, 5-Methyl-bicyclo-[2.2.1]-hept-2-en-5-yl, 5-Methyl-bicyclo-[2.2.2]-oct-2-en-5-yl, 2-Methyl-bicyclo-[4.2.2]-dec-7-en-2-yl, 2-Methyl-bicyclo-[4.3.0]-non-7-en-1-yl, 2-Methyl-bicyclo-[4.4.0]-dec-3-en-1-yl, 2-Methyl-bicyclo-[4.1.0]-hept-3-en-1-yl;
Aryl: Phenyl oder Naphthyl.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung gegen Schadpilze sind Verbindungen der Formel I bevorzugt, in denen R¹ für eine in 1-Position verzweigte Bicycloalkylgruppe oder Bicycloalkenylgruppe stehen.

Außerdem werden Verbindungen bevorzugt, in denen R² für Halogen, Alkyl oder Alkoxy steht.

Daneben werden Verbindungen I bevorzugt, in denen R² für Alkyl, insbesondere Methyl, steht.

Desweiteren werden Verbindungen I bevorzugt, in denen R² für Fluor oder Chlor steht.

Außerdem werden Verbindungen bevorzugt, in denen R³ für Alkyl Halogenalkyl, Alkoxy oder Halogenalkoxy steht.

Daneben werden Verbindungen I bevorzugt, in denen R³ für Alkyl, Halogenalkyl oder Halogenalkoxy steht.

Desweiteren werden Verbindungen I bevorzugt, in denen R³ für Methyl und Trifluormethyl steht.

Bevorzugt werden außerdem Verbindungen I, in denen R³ für Chlor steht.

Weitere bevorzugte Verbindungen I sind solche, in denen R⁴ für die folgenden Gruppen steht:
C₁-C₆-Alkyl oder C₂-C₆-Alkenyl, wobei diese Gruppen partiell oder vollständig halogeniert sein können und/oder einen dar folgenden Reste tragen können: C₁-C₄-Alkoxy, C₁-C₄-Halogenalkoxy, C₁-C₄-Alkylthio C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl, wobei diese Gruppen ein bis drei der folgenden Reste tragen können: Halogen, C₁-C₄-Alkyl, C₁-C₄-Halogenalkyl und C₁-C₄-Alkoxy;
Aryl, welches partiell oder vollständig halogeniert sein kann und oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio.

Daneben werden Verbindungen I bevorzugt, in denen R⁴ für OR⁵ oder NR⁵R⁶ steht, wobei
- R⁵: C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy;
oder Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio; und
- R⁶: Wasserstoff oder C₁-C₆-Alkyl bedeutet.

Besonders bevorzugte Verbindungen I sind solche, in denen
- R² und R³: unabhängig voneinander für Halogen, C₁-C₄-Alkyl, C₁-C₄-Halogenalkyl, C₁-C₄-Alkoxy und C₁-C₄-Halogenalkoxy stehen und
- R⁴: C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio; oder
- R⁴: C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy; oder
- R⁴: Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio.

Daneben werden auch Verbindungen I bevorzugt, in denen die Reste R², R³ und R⁴ die folgende Bedeutung haben:
- R²: Halogen, C₁-C₄-Alkyl, C₁-C₄-Halogenalkyl, C₁-C₄-Alkoxy und C₁-C₄-Halogenalkoxy;
- R³: C₁-C₄-Alkyl, C₁-C₄-Halogenalkyl, C₁-C₄-Alkoxy und C₁-C₄-Halogenalkoxy;
- R⁴: OR⁵ oder NR⁵R⁶, wobei
- R⁵: C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy; oder Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio; und
- R⁶: Wasserstoff oder C₁-C₆-Alkyl bedeutet.

Insbesondere sind auch solche Verbindungen I bevorzugt, in denen R¹ für 2-Methyl-bicyclo-[2.2.1]-hept-2-yl, 2-Methyl-bicyclo-[2.2.2]-oct-2-yl, Bicyclo-[4.1.0]-hept-1-yl, 1-Methyl-bicyclo-[4.3.0]-non-1-yl, 1-Methyl-bicyclo-[4.4.0]-dec-1-yl, 1-Methyl-bicyclo-[4.1.0]-hept-1-yl, 2-Methyl-bicyclo-[4.3.0]-non-1-yl, 2-Methyl-bicyclo-[4.4.0]-dec-1-yl, 5-Methyl-bicyclo-[2.2.1]-hept-2-yl, 5-Methyl-bicyclo-[2.2.2]-oct-2-en-5-yl, 2-Methyl-bicyclo-[4.4.0]-dec-3-en-1-yl, 2-Methyl-bicyclo-[4.1.0]-hept-3-en-1-yl;

Weiterhin sind solche Verbindungen I bevorzugt, in denen R⁴ für OR⁵ steht, wobei R⁵ eine der folgenden Gruppen bedeutet: Methyl, Ethyl, Propyl, Isopropyl, Butyl, Isobutyl, Propenyl oder Benzyl, wobei der Benzylrest ein bis fünf Halogenatome und/oder ein bis drei der folgenden Gruppen tragen kann: C₁-C₄-Alkyl, C₁-C₄-Halogenalkyl, C₁-C₄-Alkoxy, C₁-C₄-Halogenalkoxy und C₁-C₄-Alkylthio.

Daneben sind solche Verbindungen I bevorzugt, in denen R⁴ für NR⁵R⁶ steht, wobei R⁶ für Wasserstoff oder Methyl steht und R⁵ eine der folgenden Gruppen bedeutet: Methyl, iso-Butan und Benzyl.

Im Hinblick auf ihre biologische Wirkung gegen Schädlinge sind Verbindungen der allgemeinen Formel I bevorzugt, in der die Reste die folgenden Bedeutungen, für sich allein oder in Kombination, haben:
- R¹: Wasserstoff oder C₁-C₄-Alkyl, vor allem Wasserstoff oder Methyl;
- R²: Halogen, vor allem Fluor und insbesondere Chlor;
- R³: Halogen, C₁-C₄-Alkyl oder C₁-C₄-Halogenalkyl, vor allem Halogen und insbesondere Chlor oder Fluor;
- R⁴: NR⁵R⁶, vor allem mit R⁵ in der Bedeutung C₁-C₄-Alkyl, insbesondere Methyl, und vor allem mit R⁶ in der Bedeutung Wasserstoff.

Insbesondere sind im Hinblick auf ihre Verwendung die in den anschließenden Tabellen zusammengestellten Verbindungen I bevorzugt.

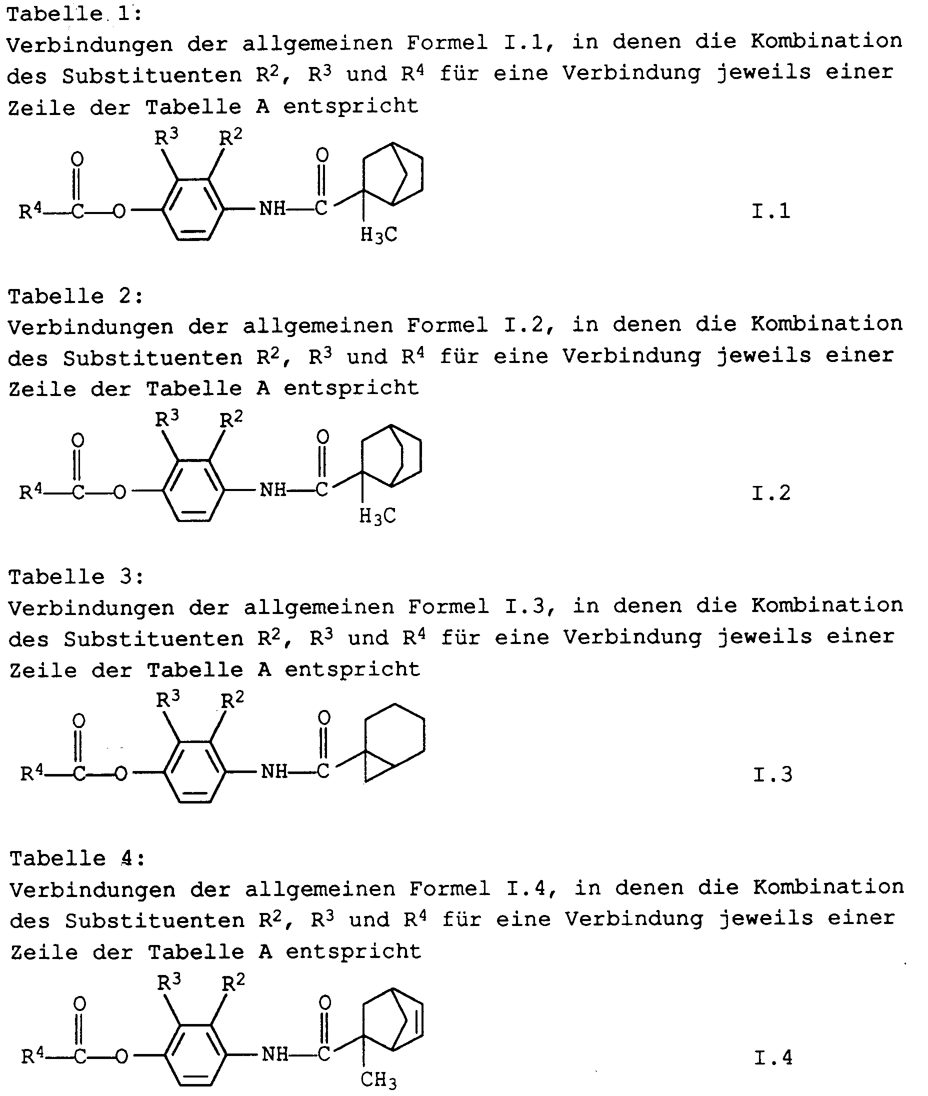

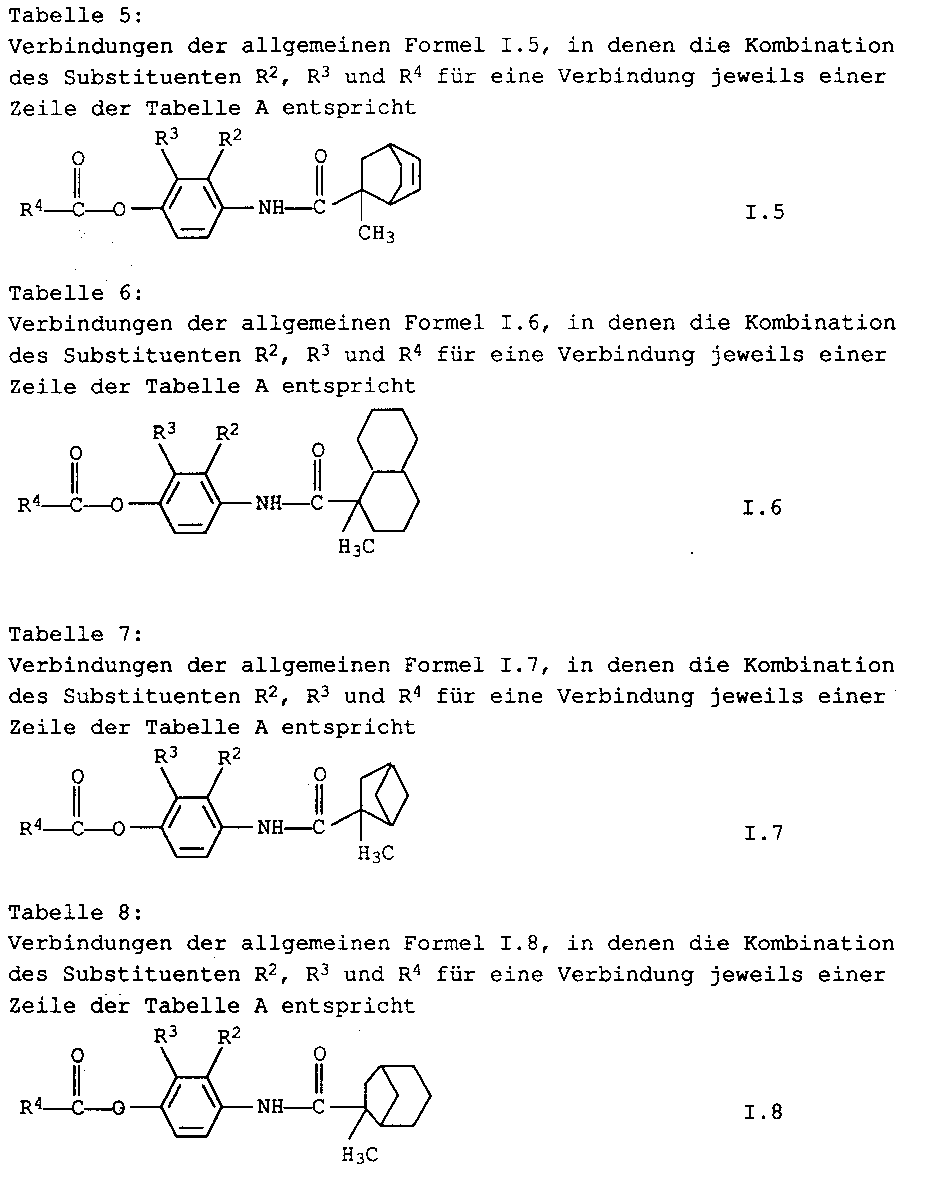

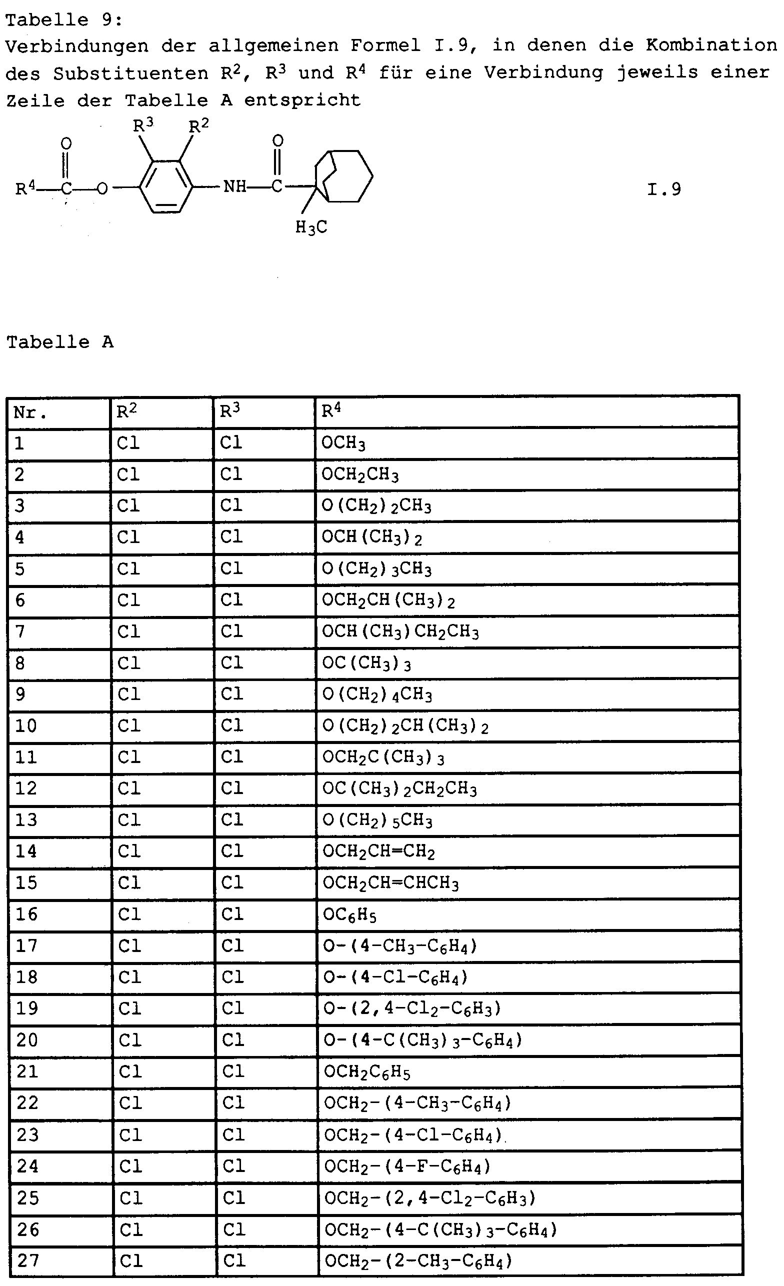

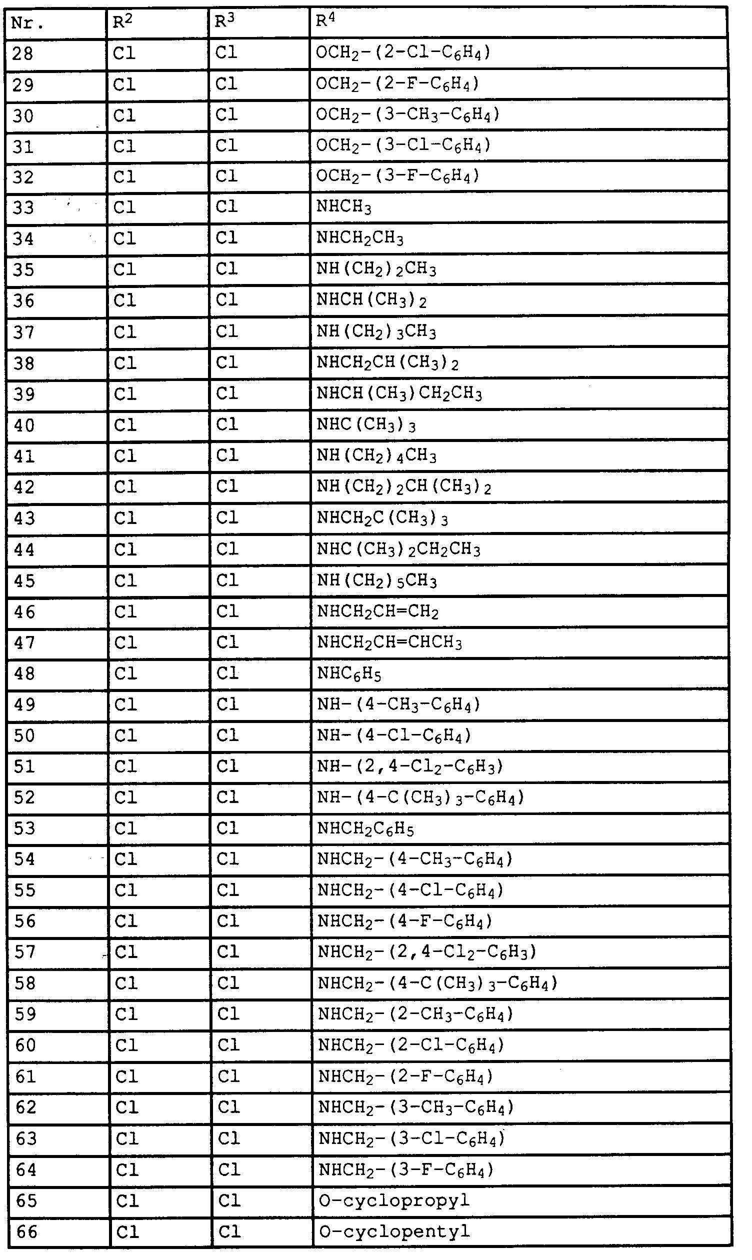

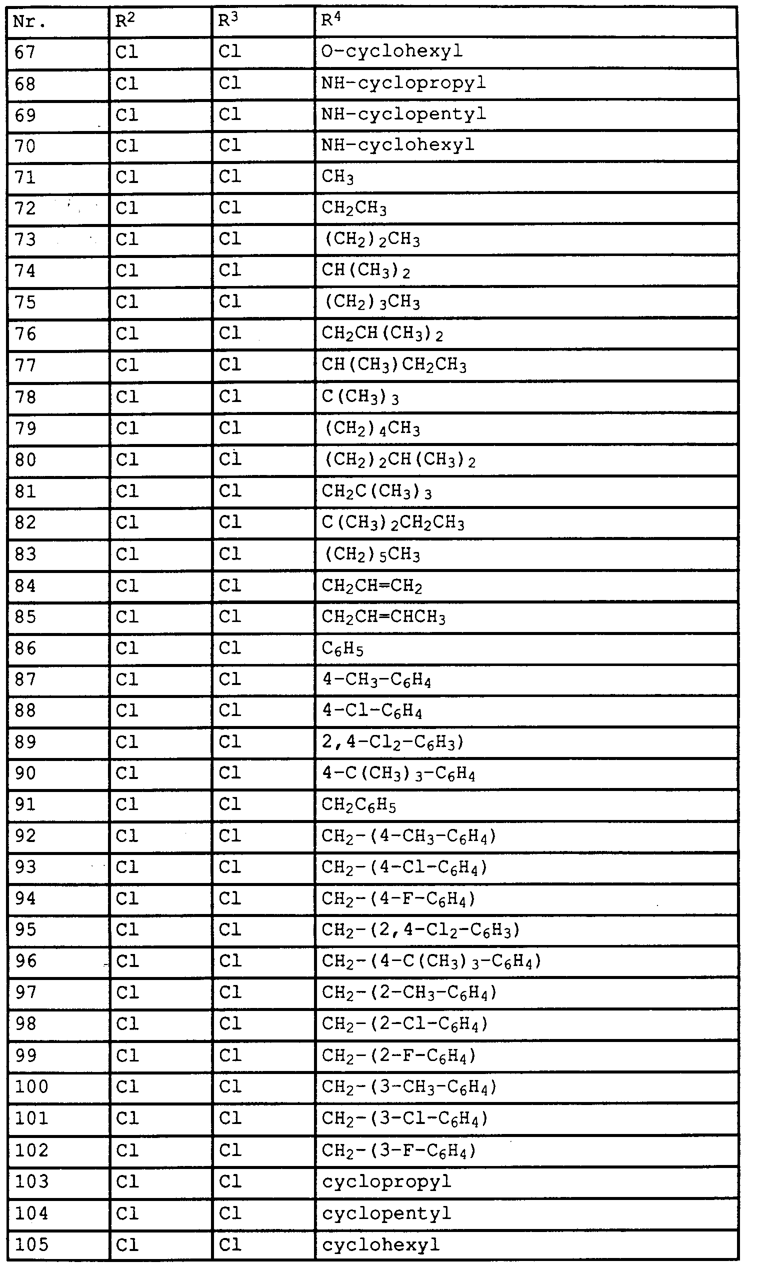

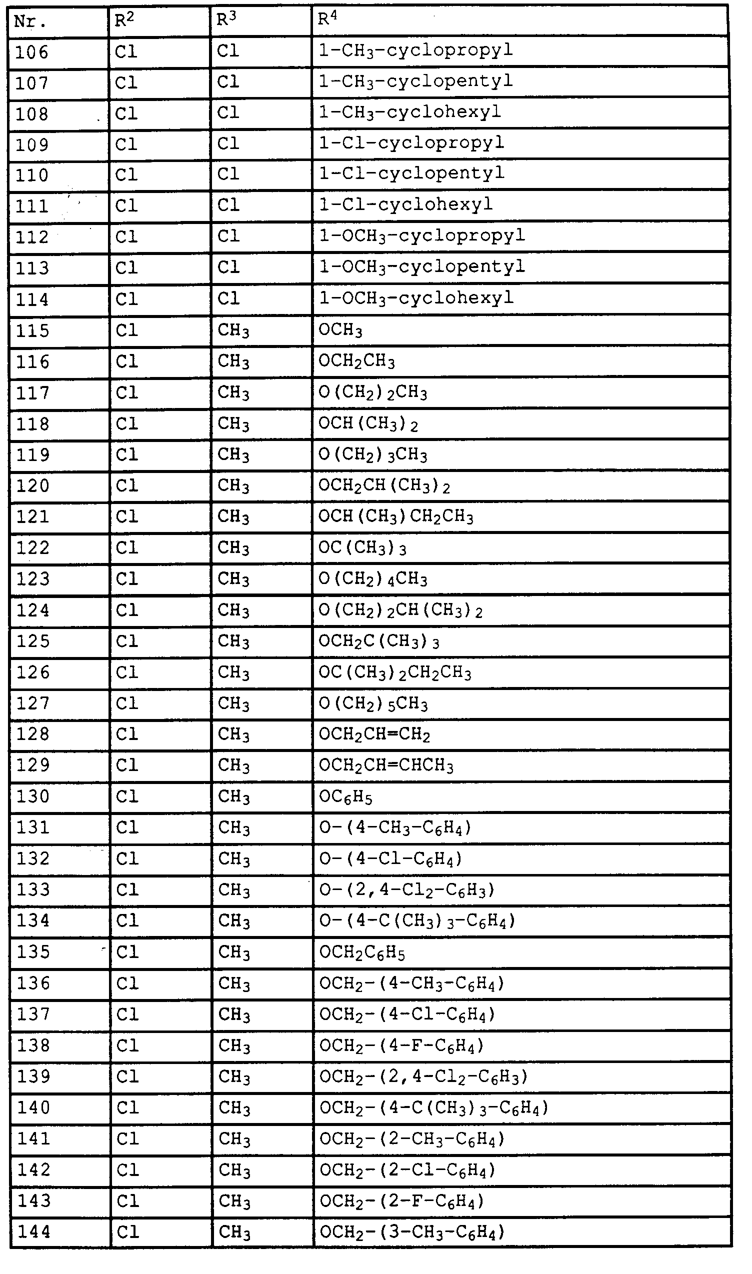

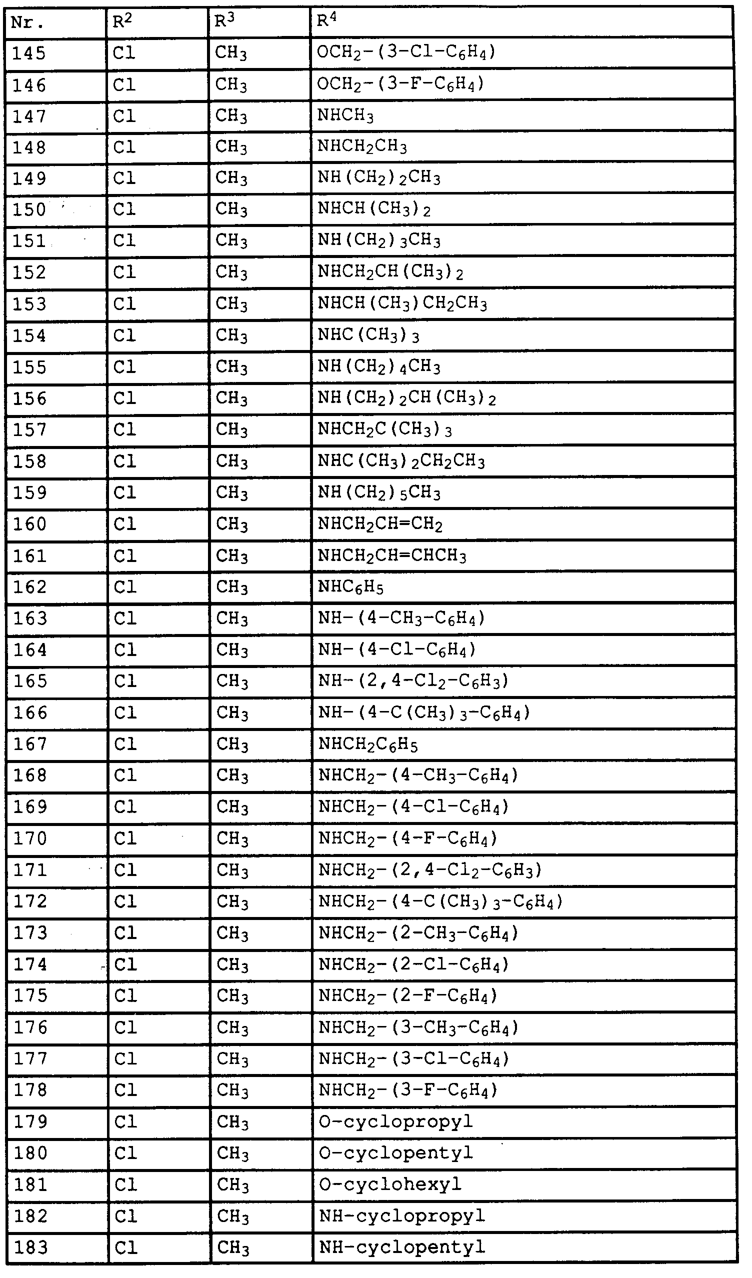

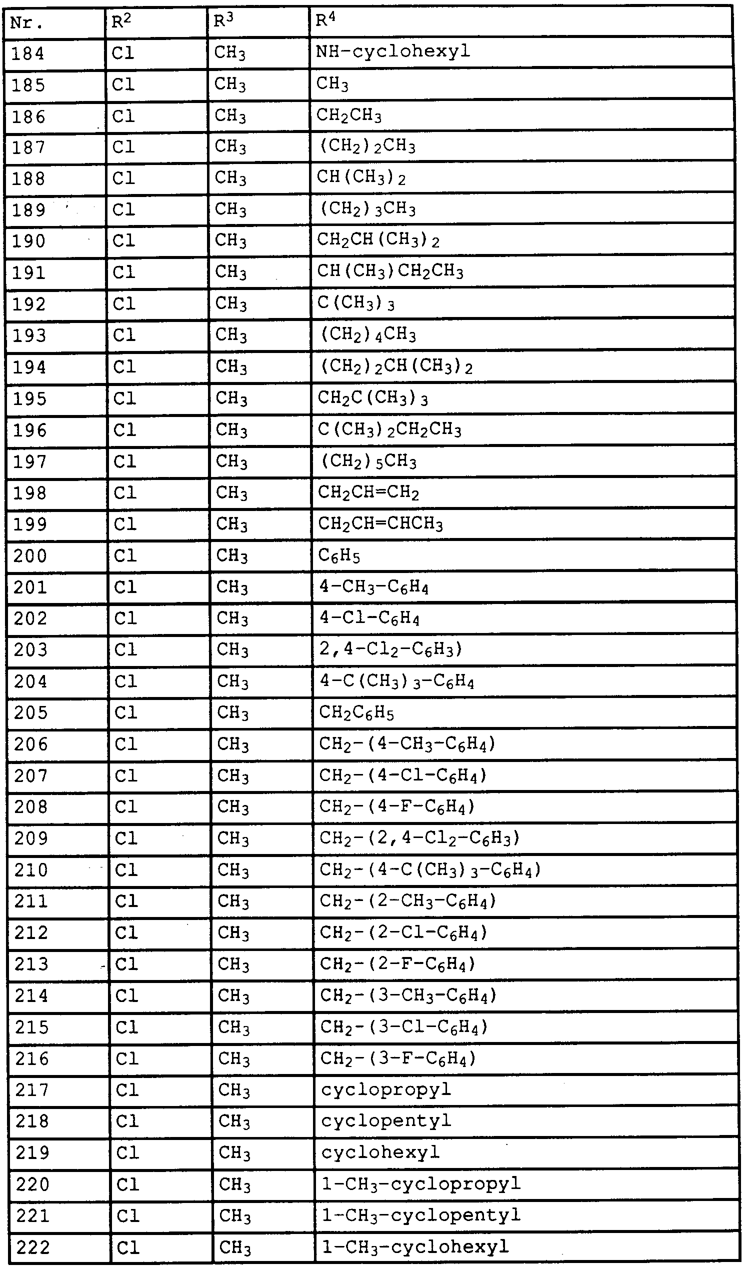

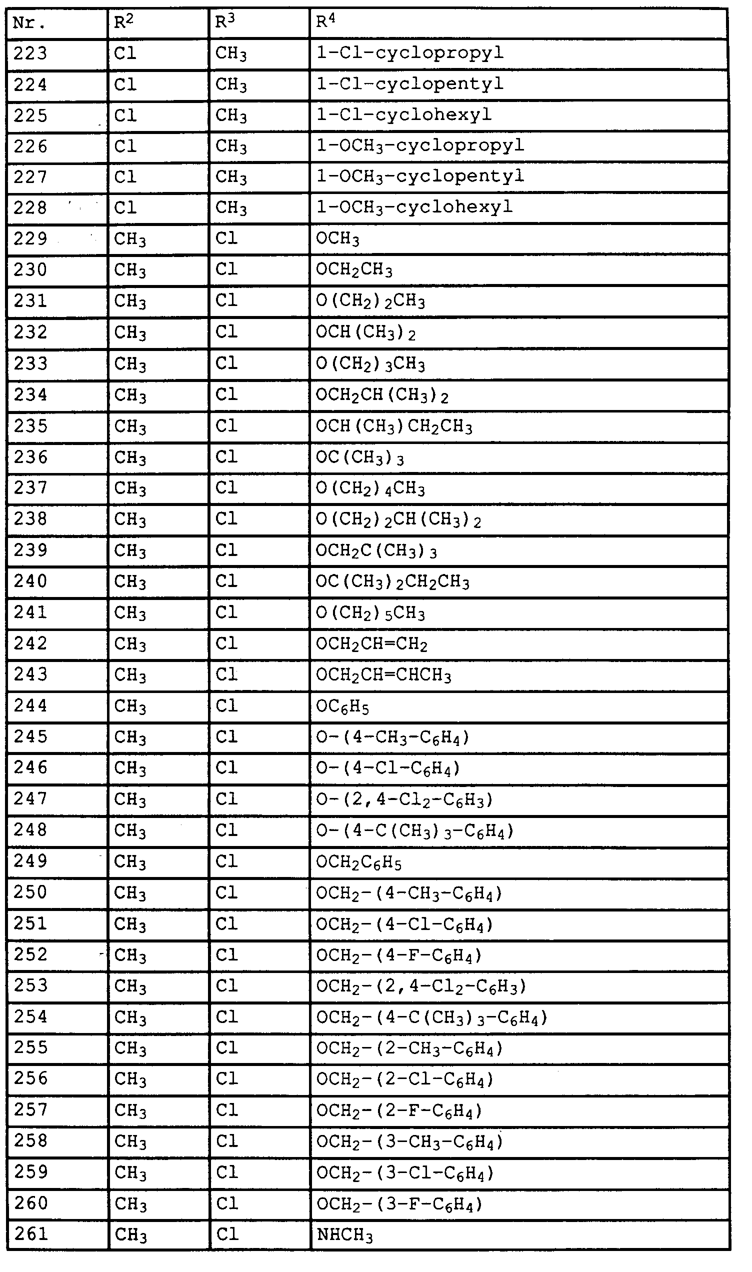

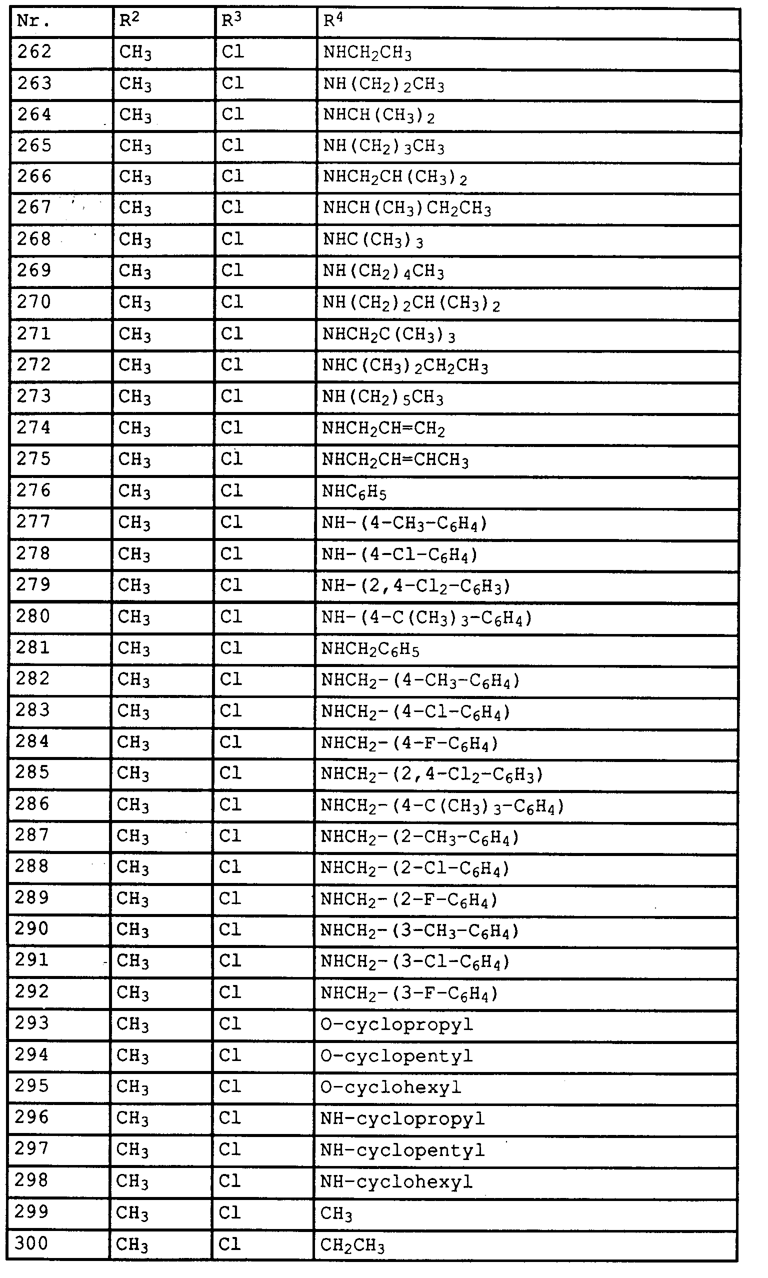

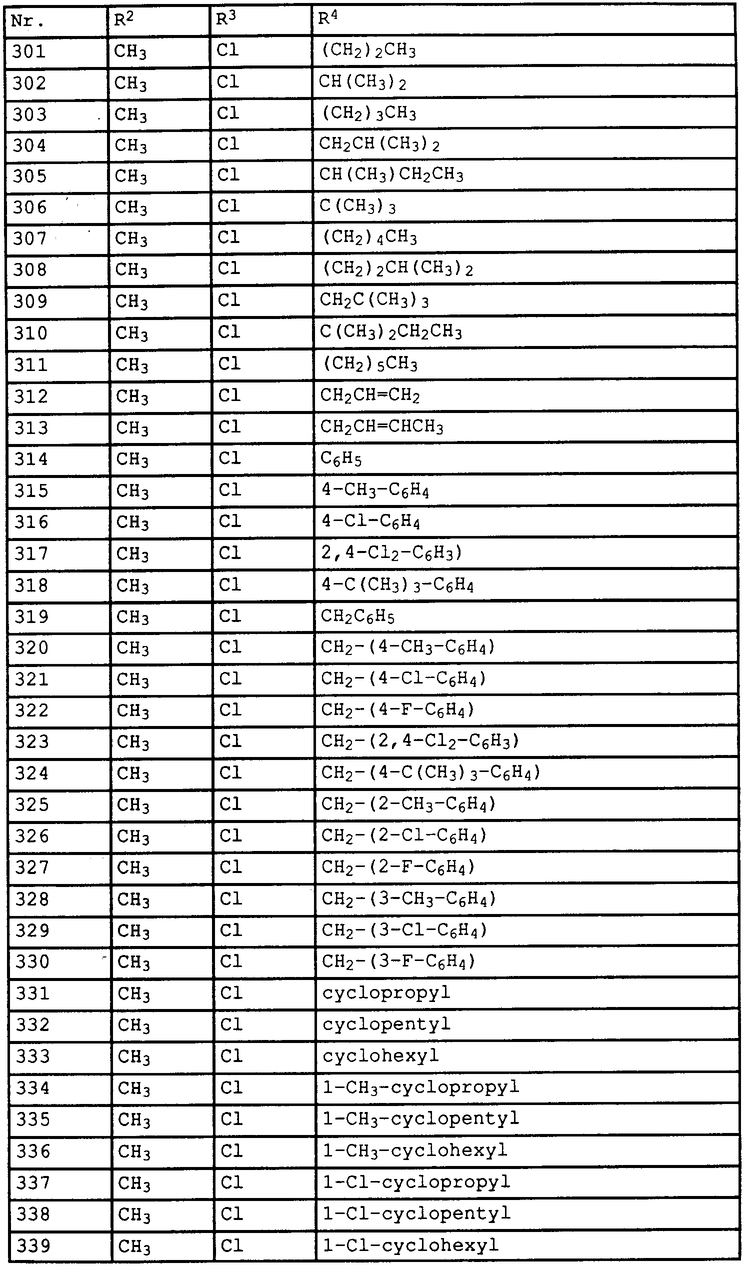

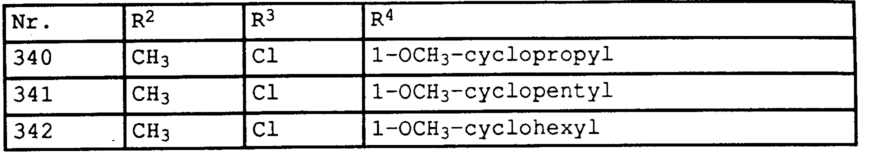

Die neuen Verbindungen der Formel I eignen sich als Fungizide.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. 1.18, und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 1.07, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1.05, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 1.10, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 1.24, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 1.13 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 1.01, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 1.11, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 1.20, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-Harnstoff-Formaldehyd-Kondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-βbis-(dimethylamino)-phosphinyl'-3-phenyl-1,2,4-triazol, 2,3-Dicyano-1,4-dithioanthrachinon, 2-Thio-1,3-dithioloβ4,5-b'chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-3-methoxycarbonyl-2-thioureido)-benzol, α-[2-(4-Chlorphenyl)ethyl]-α-(1,1-dimethylethyl)-1H,1,2,4-triazol-1-ethanol, 1-[3-(2-Chlorphenyl)-1-(4-fluorphenyl)oxiran-2-yl-methyl]-1H-1,2,4-triazol,
sowie verschiedene Fungizide, wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorhenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis,
ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus,
ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus cleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa,
ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria,
ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Eine besonders gute Wirkung zeigen die Verbindungen I gegen Schädlinge aus der Ordnung der Pflanzensauger (Homoptera).

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Praxis- und Freilandbedingungen 0,01 bis 6,0, vorzugsweise 0,1 bis 1,0 kg/ha.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Zu den Wirkstoffen können Öle verschiedenen Typs, Herbizide, Fungizide, Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Verbindungen I oder die sie enthaltenden Mittel werden bei der Bekämpfung von Schädlingen so angewendet, daß man die zu schützenden Materialien, Pflanzen, Böden oder Saatgüter mit einer wirksamen Menge einer Verbindung I oder eines sie enthaltenden Mittels behandelt.

### Synthesebeispiele

Die in den nachstehenden Synthesebeispielen wiedergegebenen Vorschriften zur Herstellung der Verbindungen I können unter Abwandlung der Ausgangsverbindungen zur Gewinnung weiterer Vertreter der Verbindungen I benutzt werden. Demgemäß hergestellte Beispiele sind in den anschließenden Tabellen mit physikalischen Daten angegeben.

### 1. Herstellung der Zwischenprodukte VI

1.1 O-(4-Amino-2,3-dichlorphenyi)-O'-(benzyl)carbonat
   Zu einer Lösung von 10 g (0,056 mol) 4-Amino-2,3-dichlorphenol in 200 ml DMF wurden unter N₂ 1,9 g (0,062 mol) 80 % Natriumhydrid zugegeben und 30 min bei RT nachgerührt. Anschließend wurde auf 0°C abgekühlt und 9,6 g (0,056 mol) Chlorameisensäurebenzylester zugetropft. Nach 2 h bei RT wurde vorsichtig 100 ml Wasser zugetropft und dreimal mit 50 ml Methylenchlorid extrahiert. Die vereinten organischen Phasen wurden zweimal mit Wasser gewaschen, getrocknet und eingeengt. Der Rückstand wurde mit Cyclohexan:Essigester=2:1 an Kieselgel chromatographiert. Ausbeute: 8,6 (49 %)
1.2 O-(4-Amino-2-chlor-3-methylphenyl)-O'-methylcarbonat
   Zu einer Lösung von 12 g (0,076 mol) 4-Amino-2,3-dichlorphenol in 200 ml DMF wurden unter N₂ 2,5 g (0,088 mol) 80 % Natriumhydrid zugegeben und 30 min bei RT nachgerührt. Anschließend wurde auf 0°C abgekühlt und 5,3 g (0,056 mol) Chlorameisensäuremethylester zugetropft. Nach 2 h bei RT wurde vorsichtig 100 ml Wasser zugetropft und dreimal mit 50 ml Methylenchlorid extrahiert. Die vereinten organischen Phasen wurden zweimal mit Wasser gewaschen, getrocknet und eingeengt. Der Rückstand wurde mit Cyclohexan:Essigester=2:1 an Kieselgel chromatographiert. Ausbeute: (63 %)
1.3 O-(4-Amino-2,3-dichlorphenyl)-N-methylcarbamat
   Zu einer Lösung von 20 g (0,112 mol) 4-Amino-2,3-dichlorphenol und 0,2 g (0,0013 mol) DBU in 130 ml Toluol wurden langsam 9,4 g (0,165 mol) Methylisocyanat zugetropft. Nach 1 h bei RT wurde auf 100 ml n-Hexan gegeben, der Niederschlag abgesaugt und mit Diisopropylether gewaschen. Ausbeute: 16,2 g (61,6 %)

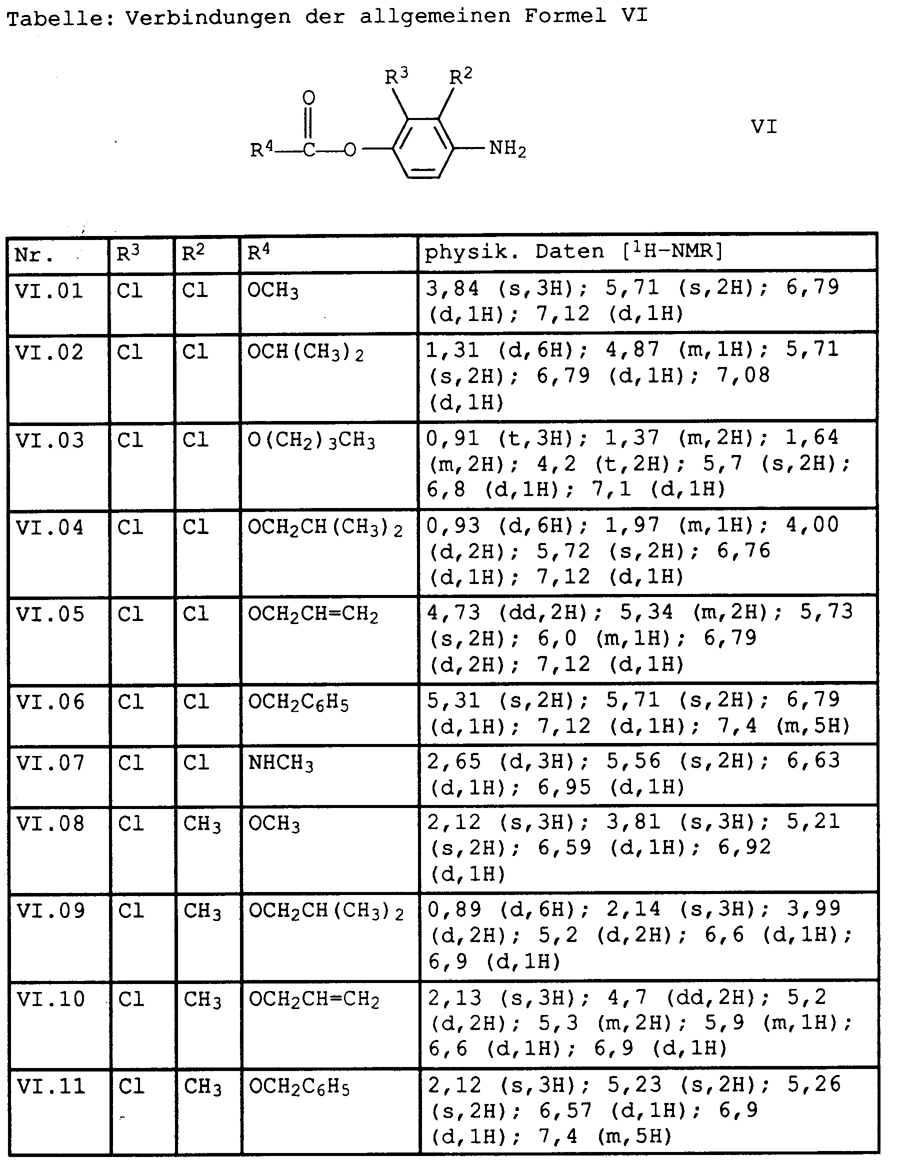

### 2. Herstellung der Wirkstoffe I

2.1 O-(4-N-Methylnorbornancarbonamid-2,3-dichlorphenyl)-O'-benzylcarbonat
   Zu einer Lösung von 1,7 g (0,0054 mol) O-(4-Amino-2,3-dichlorphenyl)-O'-benzylcarbonat in 80 ml THF wurden bei 0°C 0,6 g (0,0059 mol) Triethylamin und anschließend 1,0 g (0,0059 mol) 1-Methylnorbornancarbonsäurechlorid zugegeben. Es wurde über Nacht bei RT gerührt und anschließend auf eisgekühlte 10 %ige Salzsäure gegeben. Nach dreimaliger Extraktion mit jeweils 20 ml Methylenchlorid wurden die vereinten organischen Phasen getrocknet und eingeengt. Der verbleibende Rückstand wurde mit Cyclohexan verrührt und abgesaugt. Es verblieben 1,35 g (56 %) der Titelverbindung (Fp.: 118°).
2.2 O-(4-N-Methylnorbornancarbonamid-2,3-dichlorphenyl)-N-methylcarbamat
   Zu einer Lösung von 2 g (0,0085 mol) O-(4-Amino-2,3-dichlorphenyl)-N-methylcarbamat in 80 ml THF wurden bei 0°C 0,93 g (0,0092 mol) Triethylamin und anschließend 1,6 g (0,0092 mol) 1-Methylnorbornancarbonsäurechlorid zugegeben. Es wurde über Nacht bei RT gerührt und anschließend auf eisgekühlte 10 %ige Salzsäure gegeben. Nach dreimaliger Extraktion mit jeweils 20 ml Methylenchlorid wurden die vereinten organischen Phasen getrocknet und eingeengt. Der verbleibende Rückstand wurde mit Cyclohexan verrührt und abgesaugt. Es verblieben 1,41 g (45 %) der Titelverbindung (Fp.: 163°).
2.3 O-(4-N-(bicyclo[4.1.0]heptancarbonamid-2,3-dichlorphenyl)-O'-benzylcarbonat
   Zu einer Lösung von 1,7 g (0,0054 mol) O-(4-Amino-2,3-dichlorphenyl)-O'-methylcarbonat in 80 ml THF wurden bei 0°C 0,6 g (0,0059 mol) Triethylamin und anschließend 1,1 g (0,0092 mol) Bicyclo[4.1.0]heptancarbonsäurechlorid zugegeben. Es wurde über Nacht bei RT gerührt und anschließend auf eisgekühlte 10 %ige Salzsäure gegeben. Nach dreimaliger Extraktion mit jeweils 20 ml Methylenchlorid wurden die vereinten organischen Phasen, nacheinander mit Natriumhydrogencarbonat, Wasser gewaschen, getrocknet und eingeengt. Der verbleibende Rückstand wurde mit Cyclohexan verrührt und abgesaugt. Es verblieben 1,45 g (62 %) der Titelverbindung (Fp.: 68°).

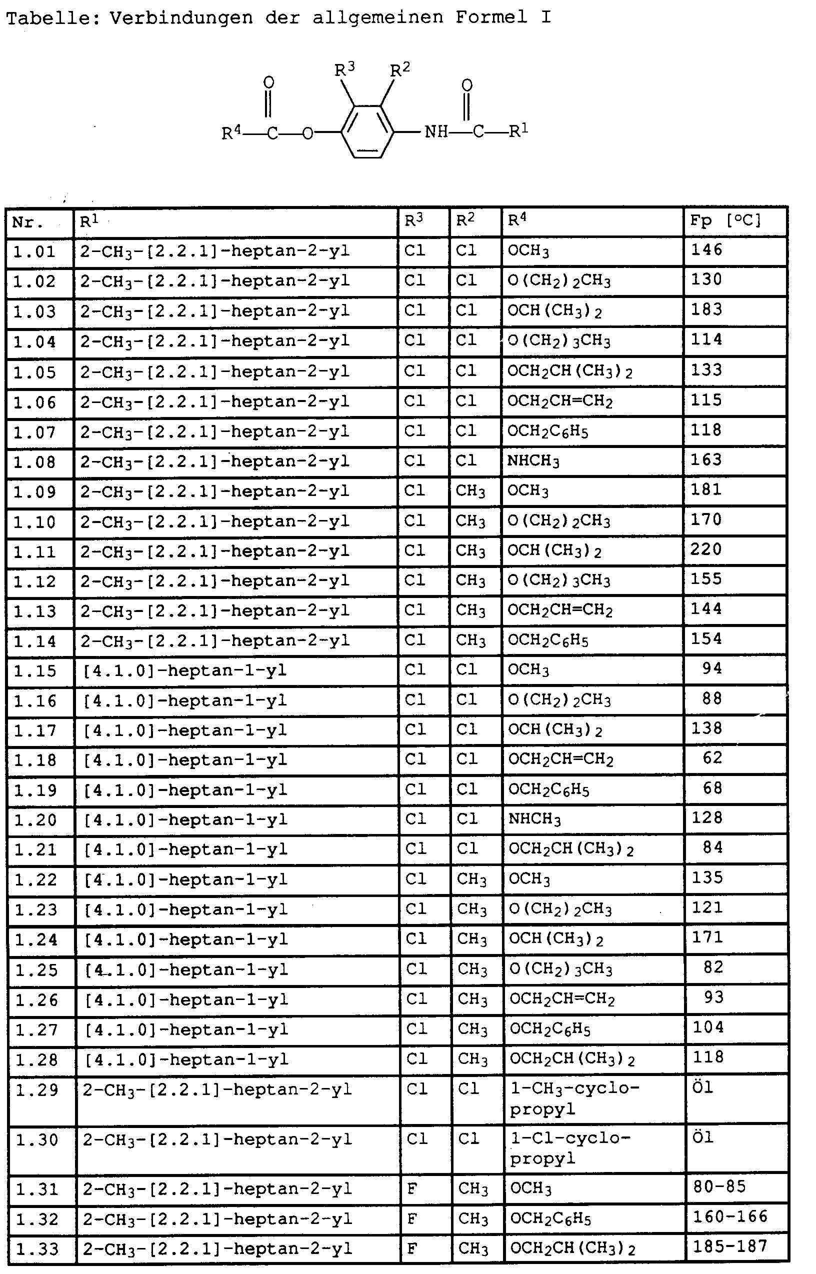

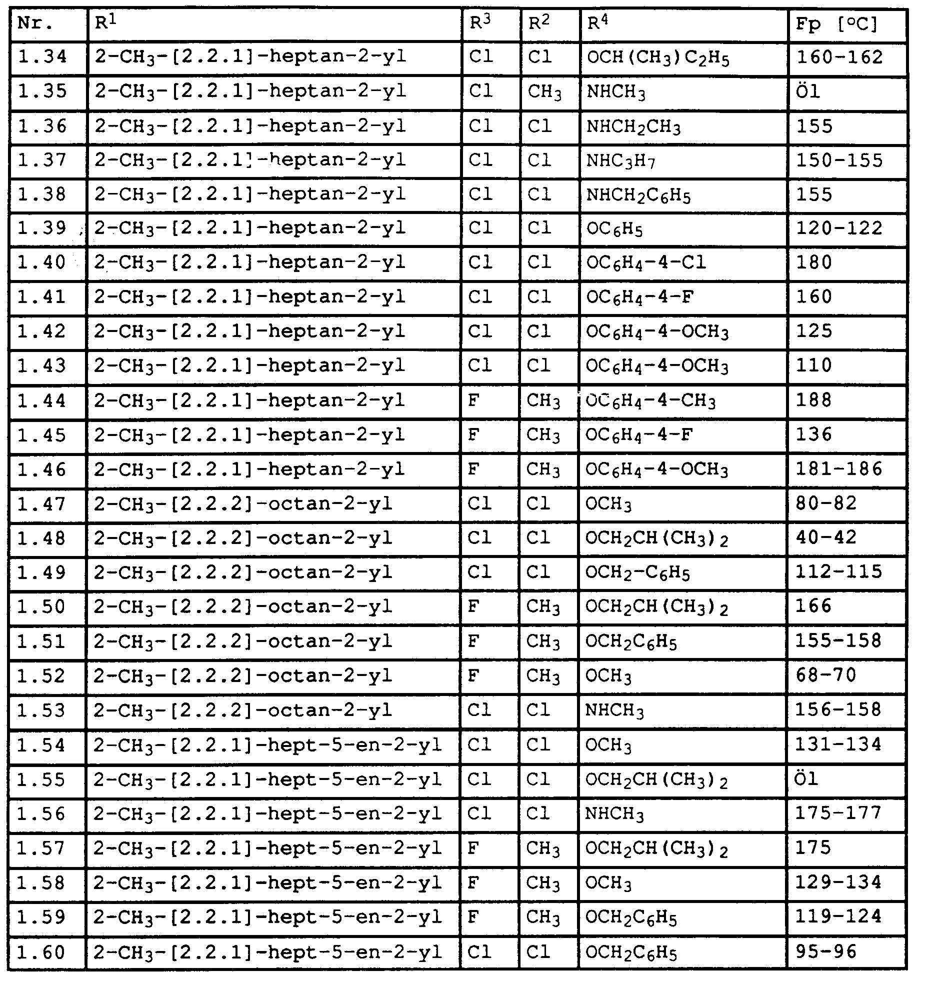

### Anwendungsbeispiele

Die fungizide Wirkung der Verbindungen der allgemeinen Formel I ließ sich durch folgende Versuche zeigen:

Die Wirkstoffe wurden als als 20 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit mit Wasser verdünnt.

### Botrytis cinera

Paprikasämlinge der Sorte "Neusiedler Ideal Elite" mit 4-5 gut entwickelten Blättern wurden mit einer wäßrigen Wirkstoffsuspension tropfnaß gespritzt. Nach dem Antrocknen des Spritzbelags wurden die Pflanzen mit einer Konidienaufschwemmung des Pilzes Botrytis cinera besprüht. Nach 5 Tagen bei 22°C - 24°C und hoher Luftfeuchtigkeit wurde der Pilz-Befall der Blätter beurteilt.

In diesem Test zeigten die mit 500 ppm der Verbindungen 1.02, 1.05, 1.07, 1.08, 1.10, 1.12, 1.13, 1.29 und 1.30 behandelten Pflanzen einen Befall von 15 % und weniger, während bei den unbehandelten Pflanzen 65 % der Blattflächen befallen waren.

### Insektizide Wirkung

Die insektizide Wirkung der Verbindungen der allgemeinen Formel I ließ sich durch folgende Versuche zeigen:

Die Wirkstoffe wurden
a) als 0,1 %ige Lösung in Aceton oder
b) als 10 %ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von (a) bzw. mit Wasser im Fall von (b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %ige Hemmung bzw. Mortalität hervorriefen (Wirkschwelle bzw. Minimalkonzentration).

## Patentansprüche

1. p-Hydroxyanilinderivate der Formel I 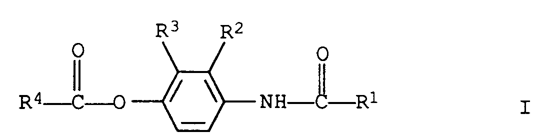 in der die Substituenten die folgende Bedeutung haben:
R¹ C₆-C₁₅-Bicyoloalkyl oder C₇-C₁₅-Bicycloalkenyl, wobei diese Gruppen partiell oder vollständig halogeniert sein können und/oder ein bis fünf der folgenden Reste tragen können: C₁-C₄-Alkyl, C₁-C₄-Halogenalkyl, C₁-C₄-Alkoxy, C₁-C₄-Halogenalkoxy und Aryl, welches partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
R² und R³ unabhängig voneinander Halogen, C₁-C₄-Alkyl, C₁-C₄-Halogenalkyl, C₁-C₄-Alkoxy und C₁-C₄-Halogenalkoxy;
R⁴ C₁-C₆-Alkyl oder C₂-C₆-Alkenyl, wobei diese Gruppen partiell oder vollständig halogeniert sein können und/oder einen der folgenden Reste tragen können:
C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl, wobei diese Gruppen ein bis drei der folgenden Reste tragen können: Halogen, C₁-C₄-Alkyl, C₁-C₄-Halogenalkyl und C₁-C₄-Alkoxy;
Aryl, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
OR⁵ oder NR⁵R⁶, wobei
R⁵ C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogen-alkyl und C₁-C₄-Alkoxy;
oder Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio; und
R⁶ Wasserstoff oder C₁-C₆-Alkyl bedeutet,
sowie deren Salze.

2. Verfahren zur Herstellung der Verbindungen I gemäß Anspruch 1, dadurch gekennzeichnet, daß man ein p-Hydroxyanilin der Formel II 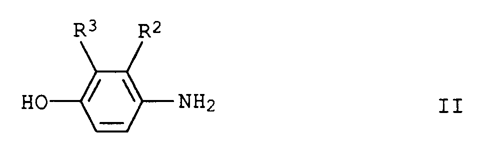 in an sich bekannter Weise in einem inerten organischen Lösungsmittel in Gegenwart einer Base mit einem Carbonylderivat der Formel III 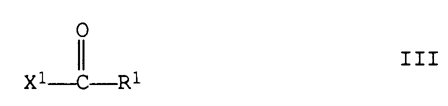 in dem X¹ für Halogen oder eine bei Acylierungsreaktionen gebräuchliche Abgangsgruppe steht, zu einem Carbonsäureamid der Formel IV 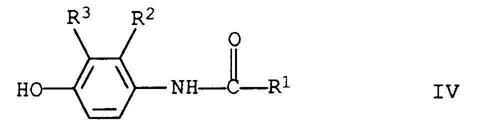 umsetzt und IV anschließend durch Umsetzung mit einem Carbonylderivat der Formel Va 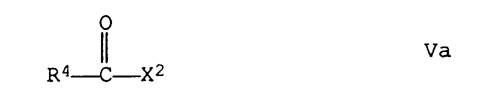 in dem X² für Halogen oder eine bei Acylierungsreaktionen gebräuchliche Abgangsgruppe steht, in I überführt.

3. Verfahren zur Herstellung der Verbindungen I gemäß Anspruch 1, in denen R⁴ für NHR⁵ steht, dadurch gekennzeichnet, daß man ein Carbonsäureamid der Formel IV gemäß Anspruch 2 in an sich bekannter Weise mit einem Isocyanat der Formel Vb
R⁵―N=C=O Vb
in I überführt.

4. Verfahren zur Herstellung der Verbindungen I gemäß Anspruch 1, dadurch gekennzeichnet, daß man ein p-Hydroxyanilin der Formel II gemäß Anspruch 2 in an sich bekannter Weise in einem inerten organischen Lösungsmittel in Gegenwart einer Base mit einem Carbonylderivat der Formel V gemäß Anspruch 2 zu einer Verbindung der Formel VI 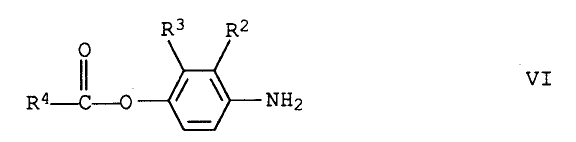 umsetzt und VI anschließend durch Umsetzung mit einem Carbonylderivat der Formel III gemäß Anspruch 2 in I überführt.

5. Zur Bekämpfung von Schadpilzen oder Schädlingen geeignetes Mittel, enthaltend übliche Zusatzstoffe und eine wirksame Menge einer Verbindung der Formel I gemäß Anspruch 1.

6. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

7. Verfahren zur Bekämpfung von Schädlingen, dadurch gekennzeichnet, daß man die Schädlinge oder die vor ihnen zu schützenden Materialien, Pflanzen, Böden oder Saatgüter mit einer wirksamen Menge einer Verbindung der allgemeinen Formel I gemäß Anspruch 1 behandelt.

8. Verwendung der Verbindungen I gemäß Anspruch 1 zur Herstellung von Mitteln gegen Schadpilze oder Schädlinge.

9. Verwendung der Verbindungen I gemäß Anspruch 1 zur Bekämpfung von Schadpilzen oder Schädlingen.

10. p-Hydroxyanilide der Formel IV 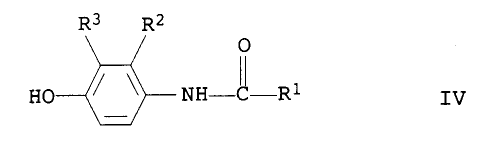 in der die Substituenten die folgenden Bedeutungen haben:
R¹ C₆-C₁₅-Bicycloalkyl oder C₇-C₁₅-Bicycloalkenyl, wobei diese Gruppen partiell oder vollständig halogeniert sein und/oder einen bis fünf der folgenden Reste tragen können: C₁-C₄-Alkyl, C₁-C₄-Halogenalkyl, C₁-C₄-Alkoxy, C₁-C₄-Halogenalkoxy und Aryl, welches partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio und
R² und R³ unabhängig voneinander Halogen, C₁-C₄-Alkyl, C₁-C₄-Halogenalkyl, C₁-C₄-Alkoxy und C₁-C₄-Halogenalkoxy.

## Claims

1. A p-hydroxyaniline derivative of the formula I 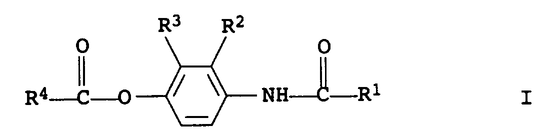 where the substituents have the following meanings:
R¹ is C₆-C₁₅-bicycloalkyl or C₇-C₁₅-bicycloalkenyl, it being possible for these groups to be partly or completely halogenated and/or to carry one to five of the following radicals: C₁-C₄-alkyl, C₁-C₄-haloalkyl, C₁-C₄-alkoxy, C₁-C₄-haloalkoxy and aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio;
R² and R³ independently of one another are halogen, C₁-C₄-alkyl, C₁-C₄-haloalkyl, C₁-C₄-alkoxy or C₁-C₄-haloalkoxy;
R⁴ is C₁-C₆-alkyl or C₂-C₆-alkenyl, it being possible for these groups to be partly or completely halogenated and/or to carry one of the following radicals: C₁-C₄-alkoxy, C₁-C₄-haloalkoxy, C₁-C₄-alkylthio, C₃-C₇-cycloalkyl, C₅-C₇-cycloalkenyl or aryl, the aromatic radicals in turn being able to carry one to three of the following groups: nitro, cyano, halogen, C₁-C₄-alkyl, C₁-C₄-haloalkyl, C₁-C₄-alkoxy, C₁-C₄-haloalkoxy and C₁-C₄-alkylthio;
C₃-C₇-cycloalkyl or C₅-C₇-cycloalkenyl, these groups being able to carry one to three of the following radicals: halogen, C₁-C₄-alkyl, C₁-C₄-haloalkyl and C₁-C₄-alkoxy;
aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio;
OR⁵ or NR⁵R⁶, where
R⁵ is C₁-C₆-alkyl or C₂-C₆-alkenyl, it being possible for these groups to be partly or completely halogenated and/or to carry one of the following radicals: C₁-C₄-alkoxy, C₁-C₄-haloalkoxy, C₁-C₄-alkylthio, C₃-C₇-cycloalkyl, C₅-C₇-cycloalkenyl or aryl, it being possible for the aromatic radicals in turn to carry one to three of the following groups: nitro, cyano, halogen, C₁-C₄-alkyl, C₁-C₄-haloalkyl, C₁-C₄-alkoxy, C₁-C₄-haloalkoxy and C₁-C₄-alkylthio;
is C₃-C₇-cycloalkyl or C₅-C₇-cycloalkenyl, it being possible for these groups to carry one to three of the following radicals: halogen, C₁-C₄-alkyl, C₁-C₄-haloalkyl and C₁-C₄-alkoxy;
or is aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio; and
R⁶ is hydrogen or C₁-C₆-alkyl,
or its salts.

2. A process for preparing the compounds I as claimed in claim 1, which comprises reacting a p-hydroxyaniline of the formula II 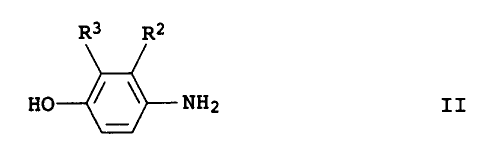 in a manner known per se in an inert organic solvent in the presence of a base with a carbonyl derivative of the formula III 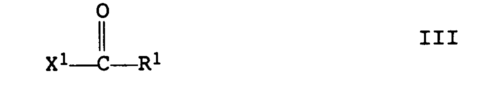 where X¹ is halogen or a leaving group which can be used in acylation reactions, to give a carboxamide of the formula IV 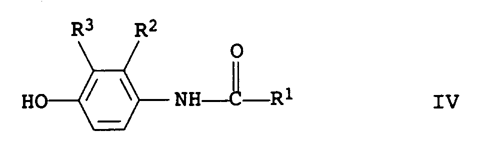 and then converting IV to I by reaction with a carbonyl derivative of the formula Va 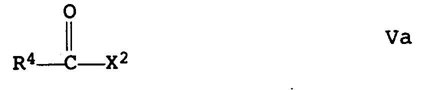 where X² is halogen or a leaving group which can be used in acylation reactions.

3. A process for preparing the compounds I as claimed in claim 1, in which R⁴ is NHR⁵, which comprises converting a carboxamide of the formula IV as in claim 2 to I in a manner known per se using an isocyanate of the formula Vb
R⁵―N=C=O Vb.

4. A process for preparing the compounds I as claimed in claim 1, which comprises reacting a p-hydroxyaniline of the formula II as in claim 2 in a manner known per se in an inert organic solvent in the presence of a base with a carbonyl derivative of the formula V as in claim 2 to give a compound of the formula VI 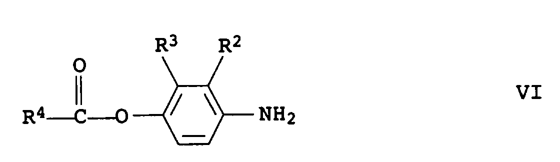 and then converting VI to I by reaction with a carbonyl derivative of the formula III as in claim 2.

5. A composition suitable for controlling harmful fungi or pests, containing customary additives and an effective amount of a compound of the formula I as claimed in claim 1.

6. A process for controlling harmful fungi, which comprises treating the harmful fungi, their environment or the plants, surfaces, materials or spaces to be kept free from them with an effective amount of a compound of the formula I as claimed in claim 1.

7. A process for controlling pests, which comprises treating the pests or the materials, plants, soils or seeds to be protected from them with an effective amount of a compound of the general formula I as claimed in claim 1.

8. The use of the compounds I as claimed in claim 1 for preparing compositions against harmful fungi or pests.

9. The use of the compounds I as claimed in claim 1 for controlling harmful fungi or pests.

10. A p-hydroxyanilide of the formula IV 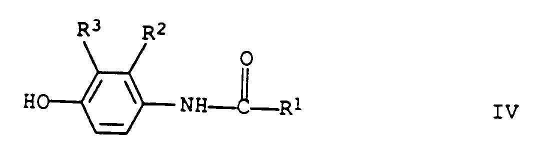 where the substituents have the following meanings:
R¹ is C₆-C₁₅-bicycloalkyl or C₇-C₁₅-bicycloalkenyl, it being possible for these groups to be partly or completely halogenated and/or to carry one to five of the following radicals: C₁-C₄-alkyl, C₁-C₄-haloalkyl, C₁-C₄-alkoxy, C₁-C₄-haloalkoxy and aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio, and
R² and R³ independently of one another are halogen, C₁-C₄-alkyl, C₁-C₄-haloalkyl, C₁-C₄-alkoxy or C₁-C₄-haloalkoxy.

## Revendications

1. Dérivés de p-hydroxyaniline de formule I 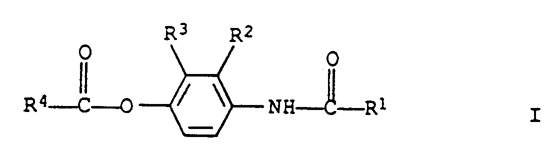 où les substituants représentent les groupes suivants:
R¹ bicycloalkyle en C₆-C₁₅ ou bicycloalcényle en C₇-C₁₅, tandis que ces groupes peuvent être partiellement ou totalement halogénés et/ou peuvent porter un à cinq des restes suivants: alkyle en C₁-C₄, halogénoalkyle en C₁-C₄, alcoxy en C₁-C₄, halogénoalcoxy en C₁-C₄ et aryle, qui peut être partiellement ou totalement halogéné et/ou peut porter un à trois des restes suivants: nitro, cyano, alkyle en C₁-C₄, halogénoalkyle en C₁-C₄, alcoxy en C₁-C₄, halogénoalcoxy en C₁-C₄ et alkylthio en C₁-C₄;
R² et R³, indépendamment l'un de l'autre, halogéno, alkyle en C₁-C₄, halogénoalkyle en C₁-C₄, alcoxy en C₁-C₄ et halogénoalkyle en C₁-C₄;
R⁴ alkyle en C₁-C₆ ou alcényle en C₂-C₆, tandis que ces groupes peuvent être partiellement ou totalement halogénés et/ou peuvent porter l'un des restes suivants: alcoxy en C₁-C₄, halogénoalcoxy en C₁-C₄, alkylthio en C₁-C₄, cycloalkyle en C₃-C₇, cycloalcényle en C₅-C₇ ou aryle, les restes aromatiques pouvant à leur tour porter un à trois des groupes suivants: nitro, cyano, halogéno, alkyle en C₁-C₄, halogénoalkyle en C₁-C₄, alcoxy en C₁-C₄, halogénoalcoxy en C₁-C₄ et alkylthio en C₁-C₄;
cycloalkyle en C₃-C₇ ou cycloalcényle en C₅-C₇, tandis que ces groupes peuvent porter un à trois des groupes suivants: halogéno, alkyle en C₁-C₄, halogénoalkyle en C₁-C₄ et alcoxy en C₁-C₄;
aryle, qui peut être partiellement ou totalement halogéné et/ou peut porter un à trois des restes suivants: nitro, cyano, alkyle en C₁-C₄, halogénoalkyle en C₁-C₄, alcoxy en C₁-C₄, halogénoalcoxy en C₁-C₄ et alkylthio en C₁-C₄;
OR⁵ ou NR⁵R⁶, tandis que
R⁵ représente un groupe alkyle en C₁-C₆ ou alcényle en C₂-C₆, tandis que ces groupes peuvent être partiellement ou totalement halogénés et/ou peuvent porter un des restes suivants: alcoxy en C₁-C₄, halogénoalcoxy en C₁-C₄, alkylthio en C₁-C₄, cycloalkyle en C₃-C₇, cycloalcényle en C₅-C₇ ou aryle, les restes aromatiques pouvant quant à eux porter un à trois des groupes suivants: nitro, cyano, halogéno, alkyle en C₁-C₄, halogénoalkyle en C₁-C₄, alcoxy en C₁-C₄, halogénoalcoxy en C₁-C₄ et alkylthio en C₁-C₄;
cycloalkyle en C₃-C₇ ou cycloalcényle en C₅-C₇, tandis que ces groupes peuvent porter un à trois des restes suivants: halogéno, alkyle en C₁-C₄, halogénoalkyle en C₁-C₄ et alcoxy en C₁-C₄;
ou aryle, qui peut être partiellement ou totalement halogéné et/ou peut porter un à trois des restes suivants: nitro, cyano, alkyle en C₁-C₄, halogénoalkyle en C₁-C₄, alcoxy en C₁-C₄, halogénoalcoxy en C₁-C₄ et alkylthio en C₁-C₄; et
R⁶ représente l'hydrogène ou un groupe alkyle en C₁-C₆,
ainsi que leurs sels.

2. Procédé pour la préparation des composés I selon la revendication 1, caractérisé par le fait qu'on fait réagir une p-hydroxyaniline de formule II 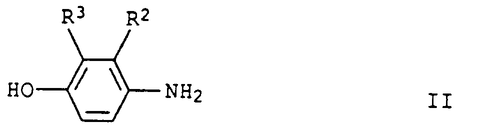 d'une manière connue en soi dans un solvant organique inerte en présence d'une base avec un dérivé de carbonyle de formule III 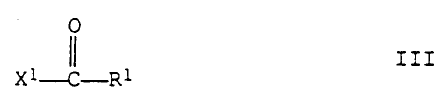 où X¹ représente un halogène ou un groupe éliminable utilisable dans des réactions d'acylation, pour former un amide d'acide carboxylique de formule IV 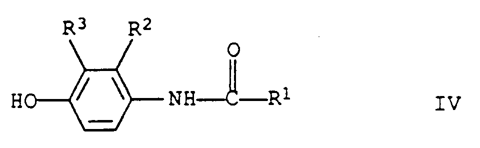 et on convertit ensuite IV en I par réaction avec un dérivé de carbonyle de formule Va 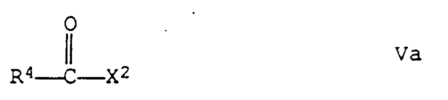 où X² représente un halogène ou un groupe éliminable utilisable dans des réactions d'acylation.

3. Procédé pour la préparation des composés I selon la revendication 1, dans lesquels R⁴ désigne NHR⁵, caractérisé par le fait qu'on convertit en I un amide d'acide carboxylique de formule IV selon la revendication 2 d'une manière connue en soi avec un isocyanate de formule Vb
R⁵―N=C=O Vb

4. Procédé pour la préparation des composés I selon la revendication 1, caractérisé par le fait qu'on fait réagir une p-hydroxyaniline de formule II selon la revendication 2 d'une manière connue en soi dans un solvant organique inerte en présence d'une base avec un dérivé de carbonyle de formule V selon la revendication 2 pour former un composé de formule VI 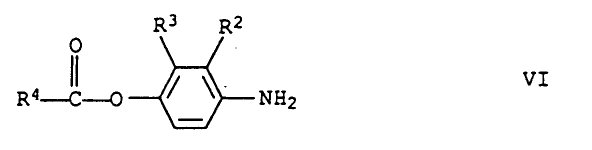 et ensuite on transforme VI en I par réaction avec un dérivé de carbonyle de formule III selon la revendication 2.

5. Agent approprié pour la lutte contre les champignons nuisibles ou les parasites, contenant des additifs classiques et une quantité efficace d'un composé de formule I selon la revendication 1.

6. Procédé pour lutter contre les champignons nuisibles, caractérisé par le fait qu'on traite les champignons nuisibles, leur biotope ou les plantes, surfaces, matières ou espaces qui ne doivent pas en comporter avec une quantité efficace d'un composé de formule I selon la revendication 1.

7. Procédé pour lutter contre les parasites, caractérisé par le fait qu'on traite les parasites ou les matières, plantes, sols ou semences à protéger contre eux avec une quantité efficace d'un composé de formule I selon la revendication 1.

8. Utilisation des composés I selon la revendication 1 pour la préparation d'agents de lutte contre les champignons nuisibles ou les parasites.

9. Utilisation des composés I selon la revendication 1 pour la lutte contre les champignons nuisibles ou les parasites.

10. p-hydroxyanilides de formule IV 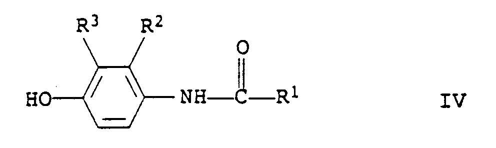 où les substituants représentent les groupes suivants:
R¹ bicycloalkyle en C₆-C₁₅ ou bicycloalcényle en C₇-C₁₅, tandis que ces groupes peuvent être partiellement ou totalement halogénés et/ou peuvent porter un à cinq des restes suivants: alkyle en C₁-C₄, halogénoalkyle en C₁-C₄, alcoxy en C₁-C₄, halogénoalcoxy en C₁-C₄ et aryle, qui peut être partiellement ou totalement halogéné et/ou peut porter un à trois des restes suivants: nitro, cyano, alkyle en C₁-C₄, halogénoalkyle en C₁-C₄, alcoxy en C₁-C₄, halogénoalcoxy en C₁-C₄ et alkylthio en C₁-C₄ et
R² et R³, indépendamment l'un de l'autre, halogéno, alkyle en C₁-C₄, halogénoalkyle en C₁-C₄, alcoxy en C₁-C₄ et halogénoalcoxy en C₁-C₄.
